# EUROPEAN PATENT APPLICATION

(11) **EP 3 583 849 A1**
(43) Date of publication of application: **25.12.2019**
(21) Application number: 19190599.1
(22) Date of filing: 04.03.2011
(51) Int. Cl.: A01N 37/00, A61K 31/20, A61K 31/232, A61P 3/06, A61P 9/00, A61K 45/06

(54) **COMPOSITIONS AND METHODS FOR TREATING AND/OR PREVENTING CARDIOVASCULAR DISEASE**

(30) Priority: 04.03.2010 US 31044310 P
(62) Divisional of application: 11751431.5
(71) Applicant: Amarin Pharmaceuticals Ireland Limited, Dublin 2 (IE)
(72) Inventor: ROWE, Jonathan, Mystic, CT Connecticut 06355 (US)
(74) Representative: Carridge, Andrew Edward

(57) **Abstract**

In various embodiments, the present invention provides pharmaceutical compositions comprising fatty acids and methods for treating subjects using same.

## Description

### BACKGROUND

Cardiovascular disease is one of the leading causes of death in the United States and most European countries. It is estimated that over 70 million people in the United States alone suffer from a cardiovascular disease or disorder including but not limited to high blood pressure, coronary heart disease, dislipidemia, congestive heart failure and stroke. A need exists for improved treatments for cardiovascular-related diseases and disorders.

### SUMMARY

In various embodiments, the present invention provides pharmaceutical compositions and methods of using such compositions to increase plasma, serum and/or red blood cell (RBC) EPA levels and/or to treat or prevent cardiovascular-related diseases.

In one embodiment, the invention provides a pharmaceutical composition comprising, consisting of or consisting essentially of at least 95% by weight ethyl eicosapentaenoate (EPA-E), about 0.2% to about 0.5% by weight ethyl octadecatetraenoate (ODTA-E), about 0.05% to about 0.25% by weight ethyl nonaecapentaenoate (NDPA-E), about 0.2% to about 0.45% by weight ethyl arachidonate (AA-E), about 0.3% to about 0.5% by weight ethyl eicosatetraenoate (ETA-E), and about 0.05% to about 0.32% ethyl heneicosapentaenoate (HPA-E). In another embodiment, the composition is present in a capsule shell. In another embodiment, the composition contains substantially no or no amount of docosahexaenoic acid (DHA) or derivative thereof such as ethyl-DHA (DHA-E), for example not more than about 0.06%, about 0.05%, or about 0.04%, by weight.

In another embodiment, the invention provides a method of increasing serum, plasma and/or red blood cell (RBC) EPA levels comprising administering a composition as described herein to a subject in need of increased serum, plasma and/or RBC EPA levels. In a related embodiment, the subject has a baseline EPA plasma, serum and/or RBC level not greater than about 50 µg/g and upon administering the composition to the subject for a period of at least about 6 weeks, the subject exhibits at least a 100%, at least a 150%, at least a 200%, at least a 250%, at least 300%, at least 350% or at least 400% increase (change in EPA level divided by baseline EPA level) in plasma, serum and/or RBC EPA levels compared to baseline. In a related embodiment, the subject has a baseline EPA plasma, serum and/or RBC level not greater than about 50 µg/g. In another embodiment, the subject is provided with an amount of said composition effective to achieve said increases in EPA levels. In another embodiment, the subject is provided with about 2 g to about 4 g per day of said composition.

In another embodiment, the invention provides a method of treating a cardiovascular-related disease in a subject in need thereof, comprising administering a composition as described herein to the subject. In a related embodiment, the subject has a baseline EPA plasma, serum and/or RBC level not greater than about 50 µg/g and upon administering the composition to the subject for a period of at least about 6 weeks, the subject exhibits at least about a 100%, at least about a 150%, at least about a 200%, at least about a 250%, at least about a 300%, at least about a 350% or at least about a 400% increase in plasma, serum and/or RBC EPA levels compared to baseline. In a related embodiment, the subject has a baseline EPA plasma, serum and/or RBC level not greater than about 50 µg/g. In another embodiment, the subject is provided with about 2 g to about 4 g per day of said composition.

These and other embodiments of the present invention will be disclosed in further detail herein below.
Fig. 1 shows blood EPA levels after various EPA administrations.
Fig. 2 shows EPA increase over baseline after various EPA administrations.

### DETAILED DESCRIPTION

While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any manner. Embodiments illustrated under any heading may be combined with embodiments illustrated under any other heading.

The use of numerical values in the various quantitative values specified in this application, unless expressly indicated otherwise, are stated as approximations as though the minimum and maximum values within the stated ranges were both preceded by the word "about." Also, the disclosure of ranges is intended as a continuous range including every value between the minimum and maximum values recited as well as any ranges that can be formed by such values. Also disclosed herein are any and all ratios (and ranges of any such ratios) that can be formed by dividing a disclosed numeric value into any other disclosed numeric value. Accordingly, the skilled person will appreciate that many such ratios, ranges, and ranges of ratios can be unambiguously derived from the numerical values presented herein and in all instances such ratios, ranges, and ranges of ratios represent various embodiments of the present invention.

In one embodiment, the invention provides pharmaceutical compositions comprising eicosapentaenoic acid or a derivative thereof. In one embodiment, such compositions comprise eicosapentaenoic acid, or a pharmaceutically acceptable ester, derivative, conjugate or salt thereof, or mixtures of any of the foregoing, collectively referred to herein as "EPA." The term "pharmaceutically acceptable" in the present context means that the substance in question does not produce unacceptable toxicity to the subject or interaction with other components of the composition.

In one embodiment, the EPA comprises all-cis eicosa-5,8,11,14,17-pentaenoic acid. In another embodiment, the EPA comprises an eicosapentaenoic acid ester. In another embodiment, the EPA comprises a C₁ - C₅ alkyl ester of eicosapentaenoic acid. In another embodiment, the EPA comprises eicosapentaenoic acid ethyl ester, eicosapentaenoic acid methyl ester, eicosapentaenoic acid propyl ester, or eicosapentaenoic acid butyl ester. In another embodiment, the EPA comprises all-cis eicosa-5,8,11,14,17-pentaenoic acid ethyl ester.

In another embodiment, the EPA is in the form of ethyl-EPA, lithium EPA, mono-, di- or triglyceride EPA or any other ester or salt of EPA, or the free acid form of EPA. The EPA may also be in the form of a 2-substituted derivative or other derivative which slows down its rate of oxidation but does not otherwise change its biological action to any substantial degree.

In another embodiment, the composition is present in a dosage unit (e.g. a capsule) in an amount of about 50 mg to about 5000 mg, about 75 mg to about 2500 mg, or about 100 mg to about 1000 mg, for example about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050 mg, about 1075 mg, about 1100 mg, about 1025 mg, about 1050 mg, about 1075 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, about 1500 mg, about 1525 mg, about 1550 mg, about 1575 mg, about 1600 mg, about 1625 mg, about 1650 mg, about 1675 mg, about 1700 mg, about 1725 mg, about 1750 mg, about 1775 mg, about 1800 mg, about 1825 mg, about 1850 mg, about 1875 mg, about 1900 mg, about 1925 mg, about 1950 mg, about 1975 mg, about 2000 mg, about 2025 mg, about 2050 mg, about 2075 mg, about 2100 mg, about 2125 mg, about 2150 mg, about 2175 mg, about 2200 mg, about 2225 mg, about 2250 mg, about 2275 mg, about 2300 mg, about 2325 mg, about 2350 mg, about 2375 mg, about 2400 mg, about 2425 mg, about 2450 mg, about 2475 mg or about 2500 mg.

In another embodiment, a composition useful in accordance with the invention contains not more than about 10%, not more than about 9%, not more than about 8%, not more than about 7%, not more than about 6%, not more than about 5%, not more than about 4%, not more than about 3%, not more than about 2%, not more than about 1%, or not more than about 0.5%, by weight, docosahexaenoic acid (DHA) or derivative thereof such as ethyl-DHA, if any. In another embodiment, a composition of the invention contains substantially no DHA or ethyl-DHA. In still another embodiment, a composition useful in the present invention contains no DHA or derivative thereof such as DHA-E.

In another embodiment, EPA comprises at least 70%, at least 80%, at least 90%, at least 95%, at least 96%, at least 97%, at least 98%, at least 99%, or 100%, by weight, of all fatty acids present in a composition according to the invention.

In another embodiment, a composition useful in accordance with the invention contains less than 10%, less than 9%, less than 8%, less than 7%, less than 6%, less than 5%, less than 4%, less than 3%, less than 2%, less than 1%, less than 0.5% or less than 0.25%, by weight of the total composition or by weight of the total fatty acid content, of any fatty acid or derivative thereof other than EPA. Illustrative examples of a "fatty acid other than EPA" include linolenic acid (LA), arachidonic acid (AA), docosahexaenoic acid (DHA), alpha-linolenic acid (ALA), stearadonic acid (STA), eicosatrienoic acid (ETA) and/or docosapentaenoic acid (DPA). In another embodiment, a composition useful in accordance with the invention contains about 0.1% to about 4%, about 0.5% to about 3%, or about 1% to about 2%, by weight, of total fatty acids other than EPA and/or DHA.

In another embodiment, a composition in accordance with the invention has one or more of the following features: (a) eicosapentaenoic acid ethyl ester represents at least about 96%, at least about 97%, or at least about 98%, by weight, of all fatty acids present in the composition; (b) the composition contains not more than about 4%, not more than about 3%, or not more than about 2%, by weight, of total fatty acids other than eicosapentaenoic acid ethyl ester; (c) the composition contains not more than about 0.6%, not more than about 0.5%, or not more than about 0.4% of any individual fatty acid other than eicosapentaenoic acid ethyl ester; (d) the composition has a refractive index (20°C) of about 1 to about 2, about 1.2 to about 1.8 or about 1.4 to about 1.5; (e) the composition has a specific gravity (20 °C) of about 0.8 to about 1.0, about 0.85 to about 0.95 or about 0.9 to about 0.92; (e) the composition contains not more than about 20 ppm, not more than about 15 ppm or not more than about 10 ppm heavy metals, (f) the composition contains not more than about 5 ppm, not more than about 4 ppm, not more than about 3 ppm, or not more than about 2 ppm arsenic, and/or (g) the composition has a peroxide value of not more than about 5 meq/kg, not more than about 4 meq/kg, not more than about 3 meq/kg, or not more than about 2 meq/kg.

In another embodiment, the invention provides a composition comprising, consisting essentially of, or consisting of at least 95%, 96% or 97%, by weight, ethyl eicosapentaenoate, about 0.2% to about 0.5% by weight ethyl octadecatetraenoate, about 0.05% to about 0.25% by weight ethyl nonaecapentaenoate, about 0.2% to about 0.45% by weight ethyl arachidonate, about 0.3% to about 0.5% by weight ethyl eicosatetraenoate, and about 0.05% to about 0.32% ethyl heneicosapentaenoate. Optionally, the composition contains not more than about 0.06%, about 0.05%, or about 0.04%, by weight, DHA or derivative there of such as ethyl-DHA. In one embodiment the composition contains substantially no or no amount of DHA or derivative there of such as ethyl-DHA. The composition further optionally comprises one or more antioxidants (e.g. tocopherol) or other impurities in an amount of not more than about 0.5% or not more than 0.05%. In another embodiment, the composition comprises about 0.05% to about 0.4%, for example about 0.2% by weight tocopherol. In another embodiment, about 500 mg to about 1 g of the composition is provided in a capsule shell.

In another embodiment, the invention provides a composition comprising, consisting of or consisting essentially of at least 96% by weight ethyl eicosapentaenoate, about 0.22% to about 0.4% by weight ethyl octadecatetraenoate, about 0.075% to about 0.20% by weight ethyl nonaecapentaenoate, about 0.25% to about 0.40% by weight ethyl arachidonate, about 0.3% to about 0.4% by weight ethyl eicosatetraenoate and about 0.075% to about 0.25% ethyl heneicosapentaenoate. Optionally, the composition contains not more than about 0.06%, about 0.05%, or about 0.04%, by weight, DHA or derivative there of such as ethyl-DHA. In one embodiment the composition contains substantially no or no amount of DHA or derivative there of such as ethyl-DHA. The composition further optionally comprises one or more antioxidants (*e.g.* tocopherol) or other impurities in an amount of not more than about 0.5% or not more than 0.05%. In another embodiment, the composition comprises about 0.05% to about 0.4%, for example about 0.2% by weight tocopherol. In another embodiment, the invention provides a dosage form comprising about 500 mg to about 1 g of the foregoing composition in a capsule shell.

In another embodiment, the invention provides a composition comprising, consisting of, or consisting essentially of at least 96%, 97% or 98%, by weight, ethyl eicosapentaenoate, about 0.25% to about 0.38% by weight ethyl octadecatetraenoate, about 0.10% to about 0.15% by weight ethyl nonaecapentaenoate, about 0.25% to about 0.35% by weight ethyl arachidonate, about 0.31% to about 0.38% by weight ethyl eicosatetraenoate, and about 0.08% to about 0.20% ethyl heneicosapentaenoate. Optionally, the composition contains not more than about 0.06%, about 0.05%, or about 0.04%, by weight, DHA or derivative there of such as ethyl-DHA. In one embodiment the composition contains substantially no or no amount of DHA or derivative there of such as ethyl-DHA. The composition further optionally comprises one or more antioxidants (*e.g.* tocopherol) or other impurities in an amount of not more than about 0.5% or not more than 0.05%. In another embodiment, the composition comprises about 0.05% to about 0.4%, for example about 0.2% by weight tocopherol. In another embodiment, the invention provides a dosage form comprising about 500 mg to about 1 g of the foregoing composition in a capsule shell.

In another embodiment, the invention provides a method of increasing serum, plasma and/or red blood cell (RBC) EPA levels comprising administering a composition as described herein to a subject in need of such treatment. In one embodiment, upon orally administering a composition as set forth herein to a subject for a period of at least about 5, about 10, about 15, about 20, about 25, about 30, about 35, about 40, about 42, about 45 or about 50 days, the subject exhibits at least about a 2-fold, at least about a 3-fold, at least about a 3.5-fold, at least about a 3.75-fold or at least about a 4-fold change (final absolute EPA level divided by baseline EPA level) in serum, plasma and/or RBC EPA. In one embodiment, the method comprises a step of identifying a patient in need of an increase in serum, plasma and/or red blood cell (RBC) EPA prior to said administration step. In a related embodiment, the subject has a baseline EPA plasma, serum and/or RBC level not greater than about 50 µg/g. In another embodiment, the subject is provided with about 2 g to about 4 g per day of said composition. In another embodiment, upon administering the composition to the subject as per above, the subject exhibits a decrease in DHA, AA and/or DGLA plasma, serum and/or RBC levels. In another embodiment, upon administering the composition to the subject as per above, the subject exhibits an increase in DPA plasma, serum and/or RBC levels. In still another embodiment, upon administering the composition to the subject as per above, DHA plasma, serum and/or RBC levels decrease by at least 16%, DGLA plasma, serum and/or RBC levels decrease by at least 31%, AA plasma, serum and/or RBC levels decrease by at least 20%, and/or DPA plasma, serum and/or RBC levels increase by greater than 130%.

In another embodiment, the invention provides a method of increasing serum, plasma and/or red blood cell (RBC) EPA levels comprising administering a composition as described herein to a subject in need of increased serum, plasma and/or RBC EPA levels. In a related embodiment, upon administering the composition to the subject for a period of at least about 5, about 10, about 15, about 20, about 25, about 30, about 35, about 40, about 42, about 45, or about 50 days, the subject exhibits at least about a 100%, at least about a 150%, at least about a 200%, at least about a 250%, at least about a 300%, at least about a 350% or at least about a 400% increase (change in EPA level from baseline divided by baseline EPA level) in plasma, serum and/or RBC EPA levels compared to baseline. In a related embodiment, the subject has a baseline EPA plasma, serum and/or RBC level not greater than about 50 µg/g. In another embodiment, the subject is provided with about 2 g to about 4 g per day of said composition. In another embodiment, upon administering the composition to the subject as per above, the subject exhibits a decrease in DHA, AA and/or DGLA plasma, serum and/or RBC levels. In another embodiment, upon administering the composition to the subject as per above, the subject exhibits an increase in DPA plasma, serum and/or RBC levels. In still another embodiment, upon administering the composition to the subject as per above, DHA plasma, serum and/or RBC levels decrease by at least 16%, DGLA plasma, serum and/or RBC levels decrease by at least 31%, AA plasma, serum and/or RBC levels decrease by at least 20%, and/or DPA plasma, serum and/or RBC levels increase by greater than 130%.

In a related embodiment, upon orally administering about 2 to about 4 g per day of a composition as set forth herein to a subject for a period of at least about 5, about 10, about 15, about 20, about 25, about 30, about 35, about 40, about 45 or about 50 days, the subject exhibits at least about a 10 µg/g increase, at least about a 15 µg/g increase, at least about a 20 µg/g increase, at least about a 25 µg/g increase, at least about a 30 µg/g increase, at least about a 35 µg/g increase, at least about a 40 µg/g increase, at least about a 45 µg/g increase, at least about a 50 µg/g increase, at least about a 75 µg/g increase, at least about a 100 µg/g increase, or at least about a 150 µg/g increase in serum, plasma and/or RBC EPA compared to baseline. In another embodiment, upon administering the composition to the subject as per above, the subject exhibits a decrease in DHA, AA and/or DGLA plasma, serum and/or RBC levels. In another embodiment, upon administering the composition to the subject as per above, the subject exhibits an increase in DPA plasma, serum and/or RBC levels. In still another embodiment, upon administering the composition to the subject as per above, DHA plasma, serum and/or RBC levels decrease by at least 16%, DGLA plasma, serum and/or RBC levels decrease by at least 31%, AA plasma, serum and/or RBC levels decrease by at least 20%, and/or DPA plasma, serum and/or RBC levels increase by greater than 130%.

In another embodiment, the subject has not been on an omega-3 fatty acid therapy or supplement for at least 2 weeks, 3 weeks, 4 weeks, 6 weeks or 12 weeks prior to initiating therapy as described herein.

In one embodiment, the invention provides a method for treatment and/or prevention of cardiovascular-related diseases comprising administering to a subject in need of such treatment or prevention a composition as set forth herein. The term "cardiovascular-related disease" herein refers to any disease or disorder of the heart or blood vessels (*i.e.* arteries and veins) or any symptom thereof. Non-limiting examples of cardiovascular-related disease and disorders include hypertriglyceridemia, hypercholesterolemia, mixed dyslipidemia, coronary heart disease, vascular disease, stroke, atherosclerosis, arrhythmia, hypertension, myocardial infarction, and other cardiovascular events.

The term "treatment" in relation a given disease or disorder, includes, but is not limited to, inhibiting the disease or disorder, for example, arresting the development of the disease or disorder; relieving the disease or disorder, for example, causing regression of the disease or disorder; or relieving a condition caused by or resulting from the disease or disorder, for example, relieving, preventing or treating symptoms of the disease or disorder. The term "prevention" in relation to a given disease or disorder means: preventing the onset of disease development if none had occurred, preventing the disease or disorder from occurring in a subject that may be predisposed to the disorder or disease but has not yet been diagnosed as having the disorder or disease, and/or preventing further disease/disorder development if already present.

In one embodiment, the present invention provides a method of blood lipid therapy comprising administering to a subject or subject group in need thereof a pharmaceutical composition as described herein. In another embodiment, the subject or subject group has hypertriglyceridemia, hypercholesterolemia, mixed dyslipidemia and/or very high triglycerides.

In another embodiment, the subject or subject group being treated has a baseline triglyceride level (or mean or median baseline triglyceride level in the case of a subject group), fed or fasting, of about 200 mg/dl to about 500 mg/dl. In another embodiment, the subject or subject group has a baseline LDL-C level (or mean or median baseline LDL-C level), despite statin therapy, of about 40 mg/dl to about 100 mg/dl.

In one embodiment, the subject or subject group being treated in accordance with methods of the invention is on concomitant statin therapy, for example atorvastatin, rosuvastatin or simvastatin therapy (with or without ezetimibe). In another embodiment, the subject is on concomitant stable statin therapy at time of initiation of ultra-pure EPA therapy.

In another embodiment, the subject or subject group being treated in accordance with methods of the invention has a body mass index (BMI or mean BMI) of not more than about 45 kg/m².

In another embodiment, the invention provides method of maintaining LDL control in a subject who is on stable statin therapy and requires triglyceride lowering therapy, the method comprising identifying a subject who is on stable statin therapy and requires triglyceride lowering therapy, administering to the subject a pharmaceutically acceptable composition comprising about 1 g to about 4 g of EPA per day (e.g. ultra-pure E-EPA), wherein upon administering the composition to the subject, the subject exhibits a clinically significant reduction in fasting triglycerides compared to control. In the present context, the term "clinically significant reduction in fasting triglycerides" means a reduction in triglycerides in an amount corresponding to a reduction in risk of an adverse cardiovascular event. Typically, each 10 mg/dl decline in triglycerides results in a 1.6% lower risk of death, myocardial infarction and recurrent acute coronary syndrome. See e.g. Miller et al., Impact of triglyceride level beyond low-density lipoprotein cholesterol after acute coronary syndrome in the PROVE IT-TIMI 22 trial. JACC Vol. 51, No. 7 (2008), hereby incorporated by reference herein. Therefore, in one embodiment, a "clinically significant reduction in fasting triglycerides" means a reduction of 10 mg/dl. In the present context, the term "maintaining LDL control" means no clinically significant adverse change in LDL levels during therapy.

In one embodiment, the invention provides a method of lowering triglycerides in a subject on stable statin therapy having baseline fasting triglycerides of about 200 mg/dl to about 500 mg/dl, the method comprising administering to the subject a pharmaceutical composition comprising about 1 g to about 4 g of EPA (*e.g.* ultra-pure EPA), wherein upon administering the composition to the subject daily for a period of about 12 weeks the subject exhibits at least 10%, at least 15%, at least 20%, at least 25%, at least 30%, at least 35%, at least 40%, at least 45%, at least 50%, at least 55%, at least 60%, at least 65%, at least 70%, or at least 75% lower fasting triglycerides than a control subject maintained on stable statin therapy without concomitant ultra-pure EPA for a period of about 12 weeks, wherein the control subject also has baseline fasting triglycerides of about 200 mg/dl to about 500 mg/dl. The term "stable statin therapy" herein means that the subject, subject group, control subject or control subject group in question has been taking a stable daily dose of a statin (*e.g.* atorvastatin, rosuvastatin or simvastatin) for at least 4 weeks prior to the baseline fasting triglyceride measurement (the "qualifying period"). For example, a subject or control subject on stable statin therapy would receive a constant daily (i.e. the same dose each day) statin dose for at least 4 weeks immediately prior to baseline fasting triglyceride measurement. In one embodiment, the subject's and control subject's LDL-C is maintained between about 40 mg/dl and about 100 mg/dl during the qualifying period. The subject and control subject are then continued on their stable statin dose for the 12 week period post baseline.

In one embodiment, the statin is administered to the subject and the control subject in an amount of about 1 mg to about 500 mg, about 5 mg to about 200 mg, or about 10 mg to about 100 mg, for example about 1 mg, about 2 mg, about 3 mg, about 4 mg, about 5 mg, about 6 mg, about 7 mg, about 8 mg, about 9 mg, or about 10 mg; about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 90 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, or about 500 mg. In another embodiment, the subject (and optionally the control subject) has a baseline LDL-C level, despite stable statin therapy, of about 40 mg/dl to about 100 mg/dl. In another embodiment, the subject and/or control subject has a body mass index (BMI; or mean BMI) of not more than about 45 kg/m².

In another embodiment, the invention provides a method of lowering triglycerides in a subject group on stable statin therapy having mean baseline fasting triglycerides of about 200 mg/dl to about 500 mg/dl, the method comprising administering to members of the subject group a pharmaceutical composition comprising about 1 g to about 4 g of ultra-pure EPA per day, wherein upon administering the composition to the members of the subject group daily for a period of about 12 weeks the subject group exhibits at least 10%, at least 15%, at least 20%, at least 25%, at least 30%, at least 35%, at least 40%, at least 45%, at least 50%, at least 55%, at least 60%, at least 65%, at least 70%, at least 75% lower mean fasting triglycerides than a control subject group maintained on stable statin therapy without concomitant ultra-pure EPA for a period of about 12 weeks, wherein the control subject group also has mean baseline fasting triglycerides of about 200 mg/dl to about 500 mg/dl. In a related embodiment, the stable statin therapy will be sufficient such that the subject group has a mean LDL-C level about at least about 40 mg/dl and not more than about 100 mg/dl for the 4 weeks immediately prior to the baseline fasting triglyceride measurement.

In another embodiment, the invention provides a method of lowering triglycerides in subject group on stable statin therapy and having mean baseline fasting triglyceride level of about 200 mg/dl to about 500 mg/dl, the method comprising administering to members of the subject group a pharmaceutical composition comprising about 1 g to about 4 g of ultra-pure EPA, wherein upon administering the composition to members of the subject group daily for a period of about 12 weeks the subject group exhibits (a) at least 10%, at least 15%, at least 20%, at least 25%, at least 30%, at least 35%, at least 40%, at least 45%, at least 50%, at least 55%, at least 60%, at least 65%, at least 70%, at least 75% lower mean fasting triglycerides by comparison with a control subject group maintained on stable statin therapy without concomitant ultra-pure EPA for a period of about 12 weeks, and (b) no increase in mean serum LDL-C levels compared to baseline, wherein the control subject also has mean baseline fasting triglycerides of about 200 mg/dl to about 500 mg/dl.

In another embodiment, the invention provides a method of lowering triglycerides in subject on stable statin therapy and having mean baseline fasting triglyceride level of about 200 mg/dl to about 500 mg/dl, the method comprising administering to the subject a pharmaceutical composition comprising about 1 g to about 4 g of ultra-pure EPA, wherein upon administering the composition to the subject daily for a period of about 12 weeks the subject exhibits (a) at least 10%, at least 15%, at least 20%, at least 25%, at least 30%, at least 35%, at least 40%, at least 45%, at least 50%, at least 55%, at least 60%, at least 65%, at least 70%, or at least 75% lower fasting triglycerides by comparison with a control subject maintained on stable statin therapy without concomitant ultra-pure EPA for a period of about 12 weeks and (b) no increase in serum LDL-C levels compared to baseline, wherein the control subject also has baseline fasting triglycerides of about 200 mg/dl to about 500 mg/dl.

In another embodiment, the invention provides a method of lowering triglycerides in subject group on stable statin therapy and having mean baseline fasting triglyceride level of about 200 mg/dl to about 500 mg/dl, the method comprising administering to members of the subject group a pharmaceutical composition comprising about 1 g to about 4 g of ultra-pure EPA, wherein upon administering the composition to the members of the subject group daily for a period of about 12 weeks the subject group exhibits (a) at least 10%, at least 15%, at least 20%, at least 25%, at least 30%, at least 35%, at least 40%, at least 45%, at least 50%, at least 55%, at least 60%, at least 65%, at least 70%, at least 75% lower mean fasting triglycerides and (b) at least 5%, at least 10%, at least 15%, at least 20%, at least 25%, at least 30%, at least 35%, at least 40%, at least 45% or at least 50% lower mean plasma or serum LDL-C levels by comparison with a control subject group maintained on stable statin therapy without concomitant ultra-pure EPA for a period of about 12 weeks, wherein the control subject also has mean baseline fasting triglycerides of about 200 mg/dl to about 500 mg/dl.

In another embodiment, the invention provides a method of lowering triglycerides in subject group on stable statin therapy and having mean baseline fasting triglyceride level of about 200 mg/dl to about 500 mg/dl, the method comprising administering to members of the subject group a pharmaceutical composition comprising about 1 g to about 4 g of ultra-pure EPA, wherein upon administering the composition to the members of the subject group daily for a period of about 12 weeks the subject group exhibits (a) at least 10%, at least 15%, at least 20%, at least 25%, at least 30%, at least 35%, at least 40%, at least 45%, at least 50%, at least 55%, at least 60%, at least 65%, at least 70%, at least 75% lower mean fasting triglycerides and (b) at least 5%, at least 10%, at least 15%, at least 20%, at least 25%, at least 30%, at least 35%, at least 40%, at least 45% or at least 50% lower mean plasma or serum LDL-C levels by comparison with a control subject group maintained on stable statin therapy without concomitant ultra-pure EPA for a period of about 12 weeks, wherein the control subject group also has mean baseline fasting triglycerides of about 200 mg/dl to about 500 mg/dl.

In another embodiment, the subject or subject group being treated in accordance with methods of the invention exhibits a fasting baseline absolute plasma level of free total fatty acid (or mean thereof) not greater than about 300 nmol/ml, not greater than about 250 nmol/ml, not greater than about 200 nmol/ml, not greater than about 150 nmol/ml, not greater than about 100 nmol/ml, or not greater than about 50 nmol/ml.

In another embodiment, the subject or subject group being treated in accordance with methods of the invention exhibits a fasting baseline absolute plasma level of free EPA (or mean thereof in the case of a subject group) not greater than about 0.70 nmol/ml, not greater than about 0.65 nmol/ml, not greater than about 0.60 nmol/ml, not greater than about 0.55 nmol/ml, not greater than about 0.50 nmol/ml, not greater than about 0.45 nmol/ml, or not greater than about 0.40 nmol/ml. In another embodiment, the subject or subject group being treated in accordance with methods of the invention exhibits a baseline fasting plasma level (or mean thereof) of free EPA, expressed as a percentage of total free fatty acid, of not more than about 3%, not more than about 2.5%, not more than about 2%, not more than about 1.5%, not more than about 1%, not more than about 0.75%, not more than about 0.5%, not more than about 0.25%, not more than about 0.2% or not more than about 0.15%. In one such embodiment, free plasma EPA and/or total fatty acid levels are determined prior to initiating therapy.

In another embodiment, the subject or subject group being treated in accordance with methods of the invention exhibits a fasting baseline absolute plasma level of free EPA (or mean thereof) not greater than about 1 nmol/ml, not greater than about 0.75 nmol/ml, not greater than about 0.50 nmol/ml, not greater than about 0.4 nmol/ml, not greater than about 0.35 nmol/ml, or not greater than about 0.30 nmol/ml.

In another embodiment, the subject or subject group being treated in accordance with methods of the invention exhibits a fasting baseline plasma, serum or red blood cell membrane EPA level not greater than about 150 µg/ml, not greater than about 125 µg/ml, not greater than about 100 µg/ml, not greater than about 95 µg/ml, not greater than about 75 µg/ml, not greater than about 60 µg/ml, not greater than about 50 µg/ml, not greater than about 40 µg/ml, not greater than about 30 µg/ml, or not greater than about 25 µg/ml.

In another embodiment, methods of the present invention comprise a step of measuring the subject's (or subject group's mean) baseline lipid profile prior to initiating therapy. In another embodiment, methods of the invention comprise the step of identifying a subject or subject group having one or more of the following: baseline non-HDL-C value of about 200 mg/dl to about 400 mg/dl, for example at least about 210 mg/dl, at least about 220 mg/dl, at least about 230 mg/dl, at least about 240 mg/dl, at least about 250 mg/dl, at least about 260 mg/dl, at least about 270 mg/dl, at least about 280 mg/dl, at least about 290 mg/dl, or at least about 300 mg/dl; baseline total cholesterol value of about 250 mg/dl to about 400 mg/dl, for example at least about 260 mg/dl, at least about 270 mg/dl, at least about 280 mg/dl or at least about 290 mg/dl; baseline vLDL-C value of about 140 mg/dl to about 200 mg/dl, for example at least about 150 mg/dl, at least about 160 mg/dl, at least about 170 mg/dl, at least about 180 mg/dl or at least about 190 mg/dl; baseline HDL-C value of about 10 to about 100 mg/dl, for example not more than about 90 mg/ dl not, not more than about 80 mg/dl, not more than about 70 mg/dl, not more than about 60 mg/dl, not more than about 60 mg/dl, not more than about 50 mg/dl, not more than about 40 mg/dl, not more than about 35 mg/dl, not more than about 30 mg/dl, not more than about 25 mg/dl, not more than about 20 mg/dl, or not more than about 15 mg/dl; and/or baseline LDL-C value of about 30 to about 300 mg/dl, for example not less than about 40 mg/dl, not less than about 50 mg/dl, not less than about 60 mg/dl, not less than about 70 mg/dl, not less than about 90 mg/dl or not less than about 90 mg/dl.

In a related embodiment, upon treatment in accordance with the present invention, for example over a period of about 1 to about 200 weeks, about 1 to about 100 weeks, about 1 to about 80 weeks, about 1 to about 50 weeks, about 1 to about 40 weeks, about 1 to about 20 weeks, about 1 to about 15 weeks, about 1 to about 12 weeks, about 1 to about 10 weeks, about 1 to about 5 weeks, about 1 to about 2 weeks or about 1 week, the subject or subject group exhibits one or more of the following outcomes:
(a) reduced triglyceride levels compared to baseline;
(b) reduced Apo B levels compared to baseline;
(c) increased HDL-C levels compared to baseline;
(d) no increase in LDL-C levels compared to baseline;
(e) a reduction in LDL-C levels compared to baseline;
(f) a reduction in non-HDL-C levels compared to baseline;
(g) a reduction in vLDL levels compared to baseline;
(h) an increase in apo A-I levels compared to baseline;
(i) an increase in apo A-I/apo B ratio compared to baseline;
(j) a reduction in lipoprotein a levels compared to baseline;
(k) a reduction in LDL particle number compared to baseline;
(l) a reduction in LDL size compared to baseline;
(m) a reduction in remnant-like particle cholesterol compared to baseline;
(n) a reduction in oxidized LDL compared to baseline;
(o) a reduction in fasting plasma glucose (FPG) compared to baseline;
(p) a reduction in hemoglobin A_{1c} (HbA_{1c}) compared to baseline;
(q) a reduction in homeostasis model insulin resistance compared to baseline;
(r) a reduction in lipoprotein associated phospholipase A2 compared to baseline;
(s) a reduction in intracellular adhesion molecule-1 compared to baseline;
(t) a reduction in interleukin-2 compared to baseline;
(u) a reduction in plasminogen activator inhibitor-1 compared to baseline;
(v) a reduction in high sensitivity C-reactive protein (hsCRP) compared to baseline;
(w) an increase in plasma or serum phospholipid EPA compared to baseline;
(x) an increase in red blood cell membrane EPA compared to baseline; and/or
(y) a reduction or increase in one or more of plasma, serum phospholipid and/or red blood cell content of docosahexaenoic acid (DHA), docosapentaenoic acid (DPA), arachidonic acid (AA), palmitic acid (PA), staeridonic acid (SA) or oleic acid (OA) compared to baseline.

In one embodiment, methods of the present invention comprise measuring baseline levels of one or more markers set forth in (a) - (y) above prior to dosing the subject or subject group. In another embodiment, the methods comprise administering a composition as disclosed herein to the subject after baseline levels of one or more markers set forth in (a) - (y) are determined, and subsequently taking an additional measurement of said one or more markers.

In another embodiment, upon treatment with a composition of the present invention, for example over a period of about 1 to about 200 weeks, about 1 to about 100 weeks, about 1 to about 80 weeks, about 1 to about 50 weeks, about 1 to about 40 weeks, about 1 to about 20 weeks, about 1 to about 15 weeks, about 1 to about 12 weeks, about 1 to about 10 weeks, about 1 to about 5 weeks, about 1 to about 2 weeks or about 1 week, the subject or subject group exhibits any 2 or more of, any 3 or more of, any 4 or more of, any 5 or more of, any 6 or more of, any 7 or more of, any 8 or more of, any 9 or more of, any 10 or more of, any 11 or more of, any 12 or more of, any 13 or more of, any 14 or more of, any 15 or more of, any 16 or more of, any 17 or more of, any 18 or more of, any 19 or more of, any 20 or more of, any 21 or more of, any 22 or more of, any 23 or more, any 24 or more, or all 25 of outcomes (a) - (y) described immediately above.

In another embodiment, upon treatment with a composition of the present invention, the subject or subject group exhibits one or more of the following outcomes:
(a) a reduction in triglyceride level of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(b) a less than 30% increase, less than 20% increase, less than 10% increase, less than 5% increase or no increase in non-HDL-C levels or a reduction in non-HDL-C levels of at least about 1%, at least about 3%, at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(c) an increase in HDL-C levels of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(d) a less than 30% increase, less than 20% increase, less than 10% increase, less than 5% increase or no increase in LDL-C levels or a reduction in LDL-C levels of at least about 5%, at least about 10%, at least about 15%, at least about 20%, at least about 25%, at least about 30%, at least about 35%, at least about 40%, at least about 45%, at least about 50%, at least about 55%, at least about 55% or at least about 75% (actual % change or median % change) as compared to baseline;
(e) a decrease in Apo B levels of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(f) a reduction in vLDL levels of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(g) an increase in apo A-I levels of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(h) an increase in apo A-I/apo B ratio of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(i) a reduction in lipoprotein(a) levels of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(j) a reduction in mean LDL particle number of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(k) an increase in mean LDL particle size of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(l) a reduction in remnant-like particle cholesterol of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(m) a reduction in oxidized LDL of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(n) a reduction in fasting plasma glucose (FPG) of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(o) a reduction in hemoglobin A_{1c} (HbA_{1c}) of at least about 5%, at least about 10%, at least about 15%, at least about 20%, at least about 25%, at least about 30%, at least about 35%, at least about 40%, at least about 45%, or at least about 50% (actual % change or median % change) compared to baseline;
(p) a reduction in homeostasis model index insulin resistance of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(q) a reduction in lipoprotein associated phospholipase A2 of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(r) a reduction in intracellular adhesion molecule-1 of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(s) a reduction in interleukin-2 of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(t) a reduction in plasminogen activator inhibitor-1 of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(u) a reduction in high sensitivity C-reactive protein (hsCRP) of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(v) an increase in plasma, serum phospholipids or RBC EPA of at least about 5%, at least about 10%, at least about 15%, at least about 20%, at least about 25%, at least about 30%, at least about 35%, at least about 40%, at least about 45%, at least about 50%, at least about 100%, at least about 200% or at least about 400% (actual % change or median % change) compared to baseline;
(w) an increase in plasma, serum phospholipid and/or RBC membrane EPA of at least about 5%, at least about 10%, at least about 15%, at least about 20%, at least about 25%, at least about 30%, at least about 35%, at least about 40%, at least about 45%, r at least about 50%, at least about 100%, at least about 200%, or at least about 400% (actual % change or median % change) compared to baseline;
(x) a reduction or increase in one or more of plasma, serum phospholipid and/or RBC DHA, DPA, AA, PA and/or OA of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) compared to baseline; and/or
(y) a reduction in total cholesterol of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) compared to baseline.

In one embodiment, methods of the present invention comprise measuring baseline levels of one or more markers set forth in (a) - (y) prior to dosing the subject or subject group. In another embodiment, the methods comprise administering a composition as disclosed herein to the subject after baseline levels of one or more markers set forth in (a) - (y) are determined, and subsequently taking a second measurement of the one or more markers as measured at baseline for comparison thereto.

In another embodiment, upon treatment with a composition of the present invention, for example over a period of about 1 to about 200 weeks, about 1 to about 100 weeks, about 1 to about 80 weeks, about 1 to about 50 weeks, about 1 to about 40 weeks, about 1 to about 20 weeks, about 1 to about 15 weeks, about 1 to about 12 weeks, about 1 to about 10 weeks, about 1 to about 5 weeks, about 1 to about 2 weeks or about 1 week, the subject or subject group exhibits any 2 or more of, any 3 or more of, any 4 or more of, any 5 or more of, any 6 or more of, any 7 or more of, any 8 or more of, any 9 or more of, any 10 or more of, any 11 or more of, any 12 or more of, any 13 or more of, any 14 or more of, any 15 or more of, any 16 or more of, any 17 or more of, any 18 or more of, any 19 or more of, any 20 or more of, any 21 or more of, any 22 or more of, any 23 or more of, any 24 or more of, or all 26 or more of outcomes (a) - (y) described immediately above.

Parameters (a) - (y) can be measured in accordance with any clinically acceptable methodology. For example, triglycerides, total cholesterol, HDL-C and fasting blood sugar can be sample from serum and analyzed using standard photometry techniques. VLDL-TG, LDL-C and VLDL-C can be calculated or determined using serum lipoprotein fractionation by preparative ultracentrifugation and subsequent quantitative analysis by refractometry or by analytic ultracentrifugal methodology. Apo A1, Apo B and hsCRP can be determined from serum using standard nephelometry techniques. Lipoprotein (a) can be determined from serum using standard turbidimetric immunoassay techniques. LDL particle number and particle size can be determined using nuclear magnetic resonance (NMR) spectrometry. Remnants lipoproteins and LDL-phospholipase A2 can be determined from EDTA plasma or serum and serum, respectively, using enzymatic immunoseparation techniques. Oxidized LDL, intercellular adhesion molecule-1 and interleukin-2 levels can be determined from serum using standard enzyme immunoassay techniques. These techniques are described in detail in standard textbooks, for example Tietz Fundamentals of Clinical Chemistry, 6th Ed. (Burtis, Ashwood and Borter Eds.), WB Saunders Company.

In one embodiment, subjects fast for up to 12 hours prior to blood sample collection, for example about 10 hours.

In another embodiment, the present invention provides a method of treating or preventing primary hypercholesterolemia and/or mixed dyslipidemia (Fredrickson Types IIa and IIb) in a patient in need thereof, comprising administering to the patient one or more compositions as disclosed herein. In a related embodiment, the present invention provides a method of reducing triglyceride levels in a subject or subjects when treatment with a statin or niacin extended-release monotherapy is considered inadequate (Frederickson type IV hyperlipidemia).

In another embodiment, the present invention provides a method of treating or preventing risk of recurrent nonfatal myocardial infarction in a patient with a history of myocardial infarction, comprising administering to the patient one or more compositions as disclosed herein.

In another embodiment, the present invention provides a method of slowing progression of or promoting regression of atherosclerotic disease in a patient in need thereof, comprising administering to a subject in need thereof one or more compositions as disclosed herein.

In another embodiment, the present invention provides a method of treating or preventing very high serum triglyceride levels (e.g. Types IV and V hyperlipidemia) in a patient in need thereof, comprising administering to the patient one or more compositions as disclosed herein.

In one embodiment, a composition of the invention is administered to a subject in an amount sufficient to provide a daily dose of ethyl eicosapentaenoic acid of about 1 mg to about 10,000 mg, 25 about 5000 mg, about 50 to about 3000 mg, about 75 mg to about 2500 mg, or about 100 mg to about 1000 mg, for example about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050 mg, about 1075 mg, about 1100 mg, about 1025 mg, about 1050 mg, about 1075 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, about 1500 mg, about 1525 mg, about 1550 mg, about 1575 mg, about 1600 mg, about 1625 mg, about 1650 mg, about 1675 mg, about 1700 mg, about 1725 mg, about 1750 mg, about 1775 mg, about 1800 mg, about 1825 mg, about 1850 mg, about 1875 mg, about 1900 mg, about 1925 mg, about 1950 mg, about 1975 mg, about 2000 mg, about 2025 mg, about 2050 mg, about 2075 mg, about 2100 mg, about 2125 mg, about 2150 mg, about 2175 mg, about 2200 mg, about 2225 mg, about 2250 mg, about 2275 mg, about 2300 mg, about 2325 mg, about 2350 mg, about 2375 mg, about 2400 mg, about 2425 mg, about 2450 mg, about 2475 mg or about 2500 mg.

In another embodiment, any of the methods disclosed herein are used in treatment of a subject or subjects that consume a traditional Western diet. In one embodiment, the methods of the invention include a step of identifying a subject as a Western diet consumer or prudent diet consumer and then treating the subject if the subject is deemed a Western diet consumer. The term "Western diet" herein refers generally to a typical diet consisting of, by percentage of total calories, about 45% to about 50% carbohydrate, about 35% to about 40% fat, and about 10% to about 15% protein. A Western diet may alternately or additionally be characterized by relatively high intakes of red and processed meats, sweets, refined grains, and desserts, for example more than 50%, more than 60% or more or 70% of total calories come from these sources.

In another embodiment, any of the methods disclosed herein are used in treatment of a subject or subjects that consume less than (actual or average) about 150 g, less than about 125 g, less than about 100 g, less than about 75 g, less than about 50 g, less than about 45 g, less than about 40 g, less than about 35 g, less than about 30 g, less than about 25 g, less than about 20 g or less than about 15 g of fish per day.

In another embodiment, any of the methods disclosed herein are used in treatment of a subject or subjects that consume less than (actual or average) about 10 g, less than about 9 g, less than about 8 g, less than about 7 g, less than about 6 g, less than about 5 g, less than about 4 g, less than about 3 g, less than about 2 g per day of omega-3 fatty acids from dietary sources.

In another embodiment, any of the methods disclosed herein are used in treatment of a subject or subjects that consume less than (actual or average) about 2.5 g, less than about 2 g, less than about 1.5 g, less than about 1 g, less than about 0.5 g, less than about 0.25 g, or less than about 0.2 g per day of EPA and DHA (combined) from dietary sources.

In one embodiment, a composition as described herein is administered to a subject once or twice per day. In another embodiment, 1, 2, 3 or 4 capsules, each containing about 500 mg to about 1 g of a composition as described herein, are administered to a subject daily. In another embodiment, 1 or 2 capsules, each containing about 1 g of a composition as described herein, are administered to the subject in the morning, for example between about 5 am and about 11 am, and 1 or 2 capsules, each containing about 1 g of a composition as described herein, are administered to the subject in the evening, for example between about 5 pm and about 11 pm.

In one embodiment, a subject being treated in accordance with methods of the invention is not on fibrate or nitrate therapy.

In another embodiment, compositions useful in accordance with methods of the invention are orally deliverable. The terms "orally deliverable" or "oral administration" herein include any form of delivery of a therapeutic agent or a composition thereof to a subject wherein the agent or composition is placed in the mouth of the subject, whether or not the agent or composition is swallowed. Thus "oral administration" includes buccal and sublingual as well as esophageal administration. In one embodiment, the composition is present in a capsule, for example a soft gelatin capsule.

A composition for use in accordance with the invention can be formulated as one or more dosage units. The terms "dose unit" and "dosage unit" herein refer to a portion of a pharmaceutical composition that contains an amount of a therapeutic agent suitable for a single administration to provide a therapeutic effect. Such dosage units may be administered one to a plurality (i.e. 1 to about 10, 1 to 8, 1 to 6, 1 to 4 or 1 to 2) of times per day, or as many times as needed to elicit a therapeutic response.

In another embodiment, the invention provides use of any composition described herein for treating moderate to severe hypertriglyceridemia in a subject in need thereof, comprising: providing a subject having a fasting baseline triglyceride level of about 500 mg/dl to about 1500 mg/dl and administering to the subject a pharmaceutical composition as described herein. In one embodiment, the composition comprises about 1 g to about 4 g of eicosapentaenoic acid ethyl ester, wherein the composition contains substantially no docosahexaenoic acid.

In another embodiment, the subject being treated has diabetes.

### EXAMPLES

The following examples are for illustrative purposes only and are not to be construed as limiting in an manner.

### Example 1

A single center, double blind, randomized, parallel-group, placebo controlled dose-ranging study of E-EPA in subjects with age-associated impairment (AAMI) was performed. The primary goal was to examine the effect of ethyl-EPA versus placebo on cognitive performance in subjects with AAMI as measure by the power of attention tasks in a computerized test batter over a period of 6 weeks. Secondary objectives were to:
(1) examine the effect of E-EPA versus placebo over 6 weeks on the following tests in the computerized cognitive battery: Continuity of attention tasks; Quality of working memory tasks; Quality of episodic memory tasks; Speed of attention tasks;
(2) to assess the safety and tolerability of E-EPA versus placebo from routine clinical laboratory tests, adverse events (AE) monitoring and vital signs; and
(3) assess the potential dose-effect relationship of E-EPA on the cognative endpoints by measurement of essential fatty acids in plasma and red blood cell membranes. 94 subjects were randomized.

The study plan was to enroll 96 subjects who would be randomly allocated to 1 of 4 possible treatment groups for 6 weeks, in a balanced block design (24 subjects per group), as follows:
1. 1 g ethyl-EPA daily
2. 2 g ethyl-EPA daily
3. 4 g ethyl-EPA daily
4. Placebo (paraffin oil) daily

Ethyl-EPA was provided as 500 mg soft gel capsules providing ethyl-EPA of >96% purity, 0.25% to 0.38% by weight ethyl octadecatetraenoate, 0.075% to 0.15% by weight ethyl nonaecapentaenoate, 0.25% to 0.35% by weight ethyl arachidonate, 0.3% to 0.4% by weight ethyl eicosatetraenoate (ETA-E), 0.075% to 0.15% ethyl heneicosapentaenoate and 0.2% dl-D-tocopherol as an antioxidant. Matching placebo capsules contained 467 g of liquid paraffin and 0.2% dl-□-tocopherol. The placebo group was further randomized so that an equal number of subjects (8) was allocated 1 g, 2 g or 4 g placebo. Study drug was taken twice daily (BID) as a divided dose (e.g. for the 1 g dose, 500 mg was given in the morning and a further 500 mg was given in the evening) with a light snack or meal.

The study consisted of a screening visit, a training visit and 4 study visits. At the screening visit, subjects' eligibility was determined through cognitive tests (verbal paired associated learning [PAL] subscale, vocabulary subtest, Memory Assessment Clinics Questionnaire [MAC-Q], mini mental state evaluation [MMSE] and MINI [mini international neuropsychiatirc interview; sections 1 and 2 of Diagnostic and Statistical Manual of Mental Disorders, 4th Ed. (DSM-IV) plus dysthymia]), haematology, clinical chemistry and 12-lead electrocardiogram (ECG). At the training visit, subjects were shown how to use the CDR computerized system. Subjects took study drug for 6 weeks and on Days 0, 14, 28 and 42, subjects underwent the CDR cognitive test battery.

### Inclusion Criteria

1. Written informed consent.
2. Male and female volunteers between 50 and 70 years of age.
3. Self-reported complaints of memory loss reflected in such everyday problems as difficulty remembering names of individuals following introduction, misplacing objects, difficulty remembering multiple items to be purchased or multiple tasks to be performed, problems remembering telephone numbers or postal codes and difficulty recalling information quickly or following distraction as determined by a score of 25 or higher on the MAC-Q questionnaire. Onset of memory loss was to be described as gradual without sudden worsening in recent months.
4. Possession of subjective and objective cognitive impairment with a score of at least 1 standard deviation (SD) below that of the mean for age-matched elderly population as determined by the total score of between 13 and 20 from the PAL subset of the Wechsler Memory Scale.
5. Evidence of adequate intellectual function as determined by a scaled score of at least 9 (raw score of at least 32) on the Vocabulary subtest of the Wechsler Adult Intelligence Scale (WAIS).
6. Absence of dementia as determined by a score of 24 or higher on the MMSE.
7. Non-smokers or ex-smokers for >3 months.
8. Was able to travel to the centre and judged by the Investigator as likely to be able to continue to travel for the duration of the study and comply with the logistical aspects of the study.
9. Body mass index (BMI) <29.5 kg/m².

### Exclusion Criteria

1. Unlikely or unable to comply with investigational medication dosing requirements.
2. Diagnosis of major depressive disorder, Alzheimer's or vascular dementia as defined according to the MINI / DSM-IV Text Revision (TR) criteria.
3. Past or current history of a neurological or psychiatric disorder that could have affected cognitive function.
4. Past or current history of inflammatory gastrointestinal disease such as Crohn's Disease or ulcerative colitis.
5. Constipation which required active treatment.
6. Current or previous history of cancer, excluding diagnosis of basal cell carcinoma.
7. Any history or evidence of clinically significant cardiac abnormality as measured by 12-lead ECG.
8. Any other medical condition or intercurrent illness not adequately controlled, which, in the opinion of the Investigator, may have put the subject at risk when participating in the study or may have influenced the results of the study or affected the subject's ability to take part in the study.
9. Clinically significant abnormal screening laboratory results (haematology, biochemistry) on screening or vital signs that fell outside the normal range for this population, which in the opinion of the Investigator affected the subject's suitability for the study.
10. Any changes to prescribed medication for a medical condition within 4 weeks of the baseline visit.
11. Omega-3 supplementation within 4 weeks of the baseline visit or during the study treatment period.
12. Currently taking anticoagulants or daily dose of aspirin >325 mg.
13. Cough or cold flu remedies containing opiates or antihistamines, within 2 weeks of the baseline visit or during the 6-week treatment period.
14. Known allergy to any ingredients of the study drug or placebo.

Any subject could withdraw from the study at any time at their or their legal guardian's request, or at the discretion of the investigator, if the subjects continued inclusion was not in their best interest, or in the event of a serious or unexpected AE. Every reasonable effort was made to document subject outcome and reasons for withdrawal. Any ongoing AEs were followed-up until the event had resolved, stabilised or was otherwise explained. Subjects who were withdrawn were not replaced. Subjects were assigned unique identification numbers according to a predetermined randomization list generated by Catalent Pharma Solutions and used in the drug packaging.

Study drug was administered orally BID as a divided dose with food, for 6 weeks. Subjects were randomized to 1 of 6 possible treatment groups (Table 1).

| **Table 1. Treatment Groups** | | | |
|---|---|---|---|
| Group Dose (g) | Study Drug | Dosage Form (soft gel capsule) | |
| **Group** | **Dose (g)** | **Study Drug** | **Dosage Form (soft gel capsule)** |
| Active 1 | 1 | Ethyl-EPA | 1 x 500 mg BID |
| Active 2 | 2 | Ethyl-EPA | 2 x 500 mg BID |
| Active 3 | 4 | Ethyl-EPA | 4 x 500 mg BID |
| Placebo 1 | 1 | Paraffin oil | 1 x 500 mg BID |
| Placebo 2 | 2 | Paraffin oil | 2 x 500 mg BID |
| Placebo 3 | 4 | Paraffin oil | 4 x 500 mg BID |

| | | | |
|---|---|---|---|
| BID = twice daily, ethyl-EPA = ethyl-eicosapentaenoic acid | | | |

Study drug was dispensed at Visits 3, 4 and 5; the maximum period between Visit 3 and each subsequent visit was:
- Visit 3 to Visit 4 (2 weeks ±2 days from Visit 3).
- Visit 3 to Visit 5 (4 weeks ±2 days from Visit 3).
- Visit 3 to Visit 6 (6 weeks ±2 days from Visit 3).

All treatment packs were identical in appearance, in order to maintain subject and investigator blind throughout the study. The investigator, Sponsor/clinical research organization personnel and subjects remained blinded throughout this study. The investigator was permitted to un-blind individual subjects if it was considered medically imperative. The process for breaking the blind is outlined below.

Omega-3 supplements had to be discontinued at least 4 weeks prior to the baseline visit (Visit 3). Cough and influenza remedies containing opiates or antihistamines had to be discontinued 2 weeks prior to the baseline visit (Visit 3) and were not permitted for the duration of the study.

Existing medication had to have been stable for 4 weeks prior to the baseline visit (Visit 3) and the dose maintained for the duration of the study. Where a dose change was absolutely necessary this was recorded in the electronic case report form (eCRF).

Subjects who required anticoagulant medication during the study were to be withdrawn. Psychological counseling or therapy was not permitted for the duration of the study, as these could have interfered with the outcome of the study. Unused study drug was returned to the study site. Subjects who used less than 80% of the prescribed dose were considered non-compliant.

At screening cognitive testing and suitability for the study were assessed using the Verbal Paired Associates 1 (Wechsler Memory Scale), Vocabulary Subtest of the WAIS, MAC-Q, MMSE and MINI (DSM-IV Sections 1 and 2 plus Dysthymia).

A selection of tasks from the CDR computerized cognitive assessment system were administered (Appendix 8 of protocol) at Visit 2 (training visit), Visit 3 (baseline), Visit 4 (Day 14), Visit 5 (Day 28) and Visit 6 (Day 42). Parallel forms of the tests were presented at each testing session. All tasks were computer-controlled, the information presented on high resolution monitors, and the responses recorded via a response model containing 2 buttons 1 marked 'no' the other 'yes'. Five CDR composite scores were used as the primary/secondary outcome variables.

The task titles were:

| | |
|---|---|
| • Word Presentation | • Numeric Working Memory |
| • Immediate Word Recall | • Delayed Word Recall |
| • Picture Presentation | • Word Recognition |
| • Simple Reaction Time | • Picture Recognition |
| • Digit Vigilance | • Bond-Lader Visual Analogue Scales of Mood and Alertness |
| • Choice Reaction Time | • Screen, Using the Computer Mouse |
| • Spatial Working Memory | |

To ensure consistency of approach, full training on the cognitive tests and CDR test battery was provided to study site staff and study subjects. The results of each variable were automatically recorded using the machine interface developed by CDR.

An AE was defined as any untoward medical occurrence temporally associated with the use of a medicinal product whether or not considered related to the medicinal product.

The investigator was responsible for the detection and documentation of AEs. At each visit the subject was asked about AEs by means of non-leading questions. AEs were recorded from the time a subject provided a written informed consent and deemed eligible to participate until completion of the treatment period. AEs ongoing at the end of the treatment period were followed until resolution or return to baseline or normal value or if the event was considered unrelated to study drug.

A serious adverse event (SAE) was defined as any AE at any dose that:
- resulted in death;
- was life-threatening;
- required hospitalization or prolongation of existing hospitalization;
- resulted in disability or incapacity, or
- resulted in a congenital anomaly/birth defect.

Other events were considered SAEs if they jeopardized the subject or required medical or surgical intervention to prevent one of the outcomes listed above.

Regardless of the above criteria, any AE that the Sponsor or investigator considered serious was to have been immediately reported as a SAE. Any death or SAE experienced by the patient while receiving or within 30 days of last dose of Investigational Medicinal Product must be promptly reported (within 24 hours of learning of the event) to pharmacovigilance. All AEs (including SAEs) are to be accurately recorded on the adverse event page of the subject's eCRF, beginning from first administration of Investigational Medicinal Product until 30 days after the last dose.

Blood samples for the laboratory assessments for haematology (a 5 mL blood sample) and clinical chemistry (a 10 mL blood sample) listed in Table 2, were collected at the screening visit (Visit 1). Samples were processed and analyzed by Simbec Laboratories Ltd.

**Table 2. Laboratory Assessments**

| **Clinical Chemistry** | **Haematology** |
|---|---|
| Sodium | Red blood cell count |
| Potassium | White blood cell count |
| Bicarbonate | Mean corpuscular volume |
| Urea | Mean corpuscular haemoglobin |
| Creatinine | Mean corpuscular haemoglobin concentration |
| Total bilirubin | Haemoglobin |
| Aspartate aminotransferase | Platelet count |
| Alanine aminotransferase | Neutrophils |
| Gamma glutamyl transferase | Lymphocytes |
| Total protein | Monocytes |
| Albumin Glucose | Basophils |

### Pharmacodynamic: Essential Fatty Acid (EFA) Measurements

Blood samples (10 mL) were collected at Visit 1 (screening) and at Visits 4, 5 and 6. Analysis was performed by MSR Lipid Analysis, Scottish Crop Research Institute, Dundee, UK. The screening sample acted as baseline for the EFA measurements.

Lipid was extracted from plasma, serum and RBC suspensions and converted into fatty acid methyl esters which were analysed by gas chromatography to give fatty acid profiles as micrograms fatty acid per gram of sample (µgFA/g) and normalised area percent. The CDR computerized system has been used to measure the effects of pharmaceuticals on cognitive function in a variety of clinical trials. Efficacy was assessed by a battery of cognition tests designed by CDR. Safety data were analysed by Quanticate.

### Populations analyzed included:

- Intent to Treat (ITT) Population: All randomised subjects with at least 1 visit post-baseline were included in this population, regardless of treatment actually received.
- Per Protocol Population (PP): All randomised subjects that completed the study, excluding significant protocol deviators, were defined as the Safety PP population. An Efficacy PP population was based on the Efficacy completers. The intercept of the Safety and Efficacy PP populations defined the Study PP Population.
- Safety Population: All randomised subjects that received at least 1 dose of study medication.

Summary statistics were provided for the ITT and Study PP Populations separately for all composite scores, major and supportive variables. Summary statistics were performed for both the unadjusted and difference from baseline data (i.e. the difference from the time matched predose assessments on Day 0). Summary statistics were calculated by treatment, day and time-point. The summary statistics comprised n, mean, median, SD, standard error of mean (SEM), minimum and maximum values.

Difference from baseline data for each major variable was evaluated by an Analysis of Covariance (ANCOVA) using SAS® PROC MIXED Version 8.2.

Fixed effects for treatment, day, time point, treatment by day, treatment by time point, treatment by day by time-point were fitted. Subject within treatment was fitted as a repeated effect using the repeated statement. The compound symmetry covariance structure was used. Subjects' time-matched predose assessments on Day 0 were used as a covariate in the analysis. Least squares means (LS means) were calculated for treatment by day, treatment by time-point and treatment by day by time-point interaction. This formal analysis was conducted for the ITT and Study PP Populations separately.

Safety evaluations were based on the safety population. Safety and tolerability were assessed in terms of AEs, vital signs, 12-lead ECG, clinical laboratory data, medical history, and study drug compliance. Safety and tolerability data were presented by treatment group. All safety data were listed individually by subject.

RBC and plasma EFA data were collected at baseline, Day 14, 28 and 42 and summarised by visit for each treatment group. Change from baseline and percent change from baseline were also summarised. ANCOVA comparison of ethyl-EPA dose groups and ethyl-EPA versus placebo was performed.

The sample size calculation was based on Power of Attention. Ispronicline (50 mg), a neuronal nicotinic acetylcholine receptor partial agonist, in subjects with AAMI on Day 21 of repeated dosing in an earlier study showed a benefit of 61 msec (50 mg mean=-32.54, SD = 61.22; placebo mean=28.25, SD = 49.64) to Power of Attention. Using a pooled SD, a sample size of 15 subjects per treatment arm was considered sufficient to detect a difference of 61 msec, with 80% power and 5% significance level (no adjustment for multiple testing). As there was no prior experience with the compound or mechanism of action with these cognitive measures, a sample size of 24 subjects per treatment arm was chosen as sufficient to allow for early withdrawals.

There were no changes to the conduct of the study. The following changes were made to the planned analyses: The equation to calculate Speed of Memory was changed to SPEEDMEM (speed of memory) = SPMRT (spatial working memory speed) + NWMRT (numeric working speed) + DRECRT (word recognition speed) + DPICRT (picture recognition speed).
- Subject's time-matched pre-dose assessments on Day 0 were used as a covariate in the analysis.
- Day 0 was removed from Day values in the list of ANCOVA variable values. Covariate = Baseline was changed to Covariate = Time matched predose assessments on Day 0 in the list of ANCOVA variable values.
- Day by Time-point was added to the list of model effects in SAS® code for ANCOVA model.
- F Tests table and Treatment Effects table were added to list of ANCOVA summary tables.
- ANCOVA summary tables were renumbered to follow on from ANCOVA raw outputs.
- Figures were included for Treatment, Treatment by Day, Treatment by Time-point, Treatment by Day by Time-point effects for ANCOVA LS means.
- Figures were added for ANCOVA LS means differences to placebo (95% confidence interval [CI]).
- A post-hoc analysis was performed which compared the individual placebo groups (1 g, 2 g and 4 g paraffin oil) with the corresponding ethyl-EPA dose rather than to a pooled placebo group.

Ninety-one subjects completed the study, three subjects discontinued; 2 subjects from the ethyl-EPA 2 g treatment group (1 subject due to an SAE considered unrelated to the study drug and 1 due to a protocol violation and 1 subject from the placebo 2 g group due to an AE.

For Power of Attention, there was no statistically significant effect of treatment, nor any treatment by day, treatment by time-point or treatment by day by time-point interactions. There was no LS mean difference between active treatment and placebo at any time-point. For Choice Reaction Time there were statistically significant benefits for ethyl-EPA 1 g and 2g on Day 28, and some trends for benefit for 1 and 4 g ethyl-EPA on Day 42, versus placebo; however no clear treatment-related pattern was observed.

Continuity of Attention did not show a difference between placebo and ethyl-EPA, except for an overall decrease for 2 g ethyl-EPA that was only visible in the ITT population. The subtask Digit Vigilance Targets Detected showed isolated decreases for active treatment versus placebo, but there was no obvious treatment-related pattern.

Quality of Working Memory was the only composite score that showed a statistically significant treatment by day interaction in the F-ratio. However, there were only isolated statistically significant decreases for ethyl-EPA 1 g and 2 g versus placebo on Days 14 and 28, and these were most likely to be due to chance and not treatment related.

Quality of Episodic Secondary Memory showed statistically significant decreases for ethyl-EPA versus placebo at various time-points. However, it seems unlikely to be an effect of active treatment as the unadjusted data showed pre-existing differences between the treatment groups that was most notable on Day 0 in the first assessment session. In difference from Baseline data that were calculated prior to ANCOVA analysis, these differences were no longer apparent. This suggests that the ANCOVA model fitted a strong negative correlation with the baseline values. This is often the case when the variability within subjects overlaps the variability between subjects.

Speed of Memory and the subtasks Spatial and Numeric Working Memory Speeds and Word and Picture Recognition Speed showed no differences in performance, in the F-ratio statistics, between Ethyl-EPA and placebo.

For Self-rated Alertness, there was no apparent difference in ratings between ethyl-EPA and placebo. There were isolated decreases in ratings for active treatment versus placebo that were unlikely to be compound related.

Self-rated Contentment showed statistically significant decreases in ratings for ethyl-EPA 2 g on Day 28. However, these individual decreases were not statistically significant. It is unlikely that this was a treatment-related effect as it was restricted to a single day and no other dose level showed a similar pattern on any other day. For Self-rated Calmness there was no difference in ratings between active treatment and placebo.

When the results of each ethyl-EPA dose and their corresponding placebo were compared (post-hoc analysis), it appeared that ethyl-EPA 4 g improved the subjects' reaction times in the attention tasks (Power of Attention, Simple Reaction Time and Choice Reaction Time). This was seen most clearly for Choice Reaction Time, where a pattern of gradual improvement over the assessment day for 4 g was seen. It is possible that a longer period of administration would clarify the effects of ethyl -EPA on these parameters.

EPA (shown in Table 3), DPAn-3 and EPA/AA ratio (data not shown) plasma and RBC values increased substantially from baseline to Day 42 for the AMR-101 1, 2, and 4 g treatment groups. AA, DHA and DGLA values decreased substantially from baseline (data not shown).

**Table 3. Mean (SD) EPA (Plasma and RBC (µg/g)) Change from Baseline.**

| | **Ethyl-EPA** | | | **Placebo** | | |
|---|---|---|---|---|---|---|
| | 1 g (N=23) | 2 g (N=24) | 4 g (N=24) | 1 g (N=7) | 2 g (N=8) | 4 g (N=8) |
| **Plasma** | | | | | | |
| Baseline | 48.3 (31.03) | 44.9 (25.01) | 49.1 (17.23) | 47.5 (26.41) | 42.1 (16.18) | 42.5 (11.86) |
| Day 14 | 61.2 (26.61) | 124.6 (42.25) | 207.7 (57.05) | 1.6 (24.69 | -1.2 (19.82) | 21.9 (32.91) |
| Day 28 | 60.3 (36.03) | 142.2 (46.23) | 215.2 (58.68) | 6.5 (15.46) | 1.6 (13.64) | 1.3 (14.03) |
| Day 42 | 62.0 (39.43) | 133.4 (43.34) | 204.6 (80.69) | 11.9 (26.34) | 0.4 (21.18) | 4.4 (23.32) |

| **RBC** | | | | | | |
|---|---|---|---|---|---|---|
| Baseline | 19.8 (10.85 | 18.9 (8.91) | 19.8 5.28) | 20.4 (5.77 | 19.3 (6.58) | 17.2 (4.94) |
| Day 14 | 12.3 (7.39) | 26.9 (9.15) | 39.5 (13.16) | -0.5 (6.32) | 0.0 (7.17) | 2.6 (6.73) |
| Day 28 | 14.5 (10.47) | 32.9 (10.11) | 50.2 (15.82) | 1.5 (4.16) | 0.0 (7.06 | 0.6 (4.42) |
| Day 42 | 17.6 (11.89) | 38.3 (12.46) | 52.5 (20.56) | -0.2 (5.90) | 1.0 (8.01) | -0.2 (6.97) |

As can be seen in Table 3, at the 2 g per day AMR101 dose, plasma EPA levels increased 297% after 42 days and at the 4 g per day AMR101 dose, plasma EPA levels increased by 417% compared to baseline.

Grimsgaard et al. previously published an article describing serum phospholipid levels at baseline and after 7 weeks of supplementation with 4 g per day of 90% ethyl-DHA, 4 g per day of 95% ethyl-EPA with some DHA present, or corn oil. Am. J. Clin. Nutr. 1997; 66:649-59 (1997). The complete profile of additional fatty acids and ingredients present in these compositions is unknown. After supplementation over a period of 7 weeks, subjects exhibited only a 297% increase in serum phospholipid EPA compared to the increase of 417% shown above with an inventive composition. A comparison of other changes in plasma/serum fatty acids is shown in Table 4.

**Table 4. Percent Fatty Acid Change from Baseline After Administration of 4 g Dose**

| **Fatty Acid** | **Grimsgaard** | **AMR101** |
|---|---|---|
| EPA | +297% | +417% |
| AA | -18.5% | -21.9% |
| DHA | -15.20% | -17.5% |
| DPA | +130% | +147% |
| DGLA | -30.5% | -39.4% |

Furthermore, in the Japanese Eicosapentaenoic Acid (EPA) Lipid Intervention Study (JELIS), Yokoyama et al. reported that they followed over 18,000 patients randomly assigned to received either 1800 mg of EPA composition (Epadel) with statin, or statin only with a 5-year follow-up. Lancet 2007; 369: 1090-98. After 5 years of treatment, subjects exhibited an increase in plasma EPA of only 70% (from baseline of 93 mg/L to 169 mg/L).

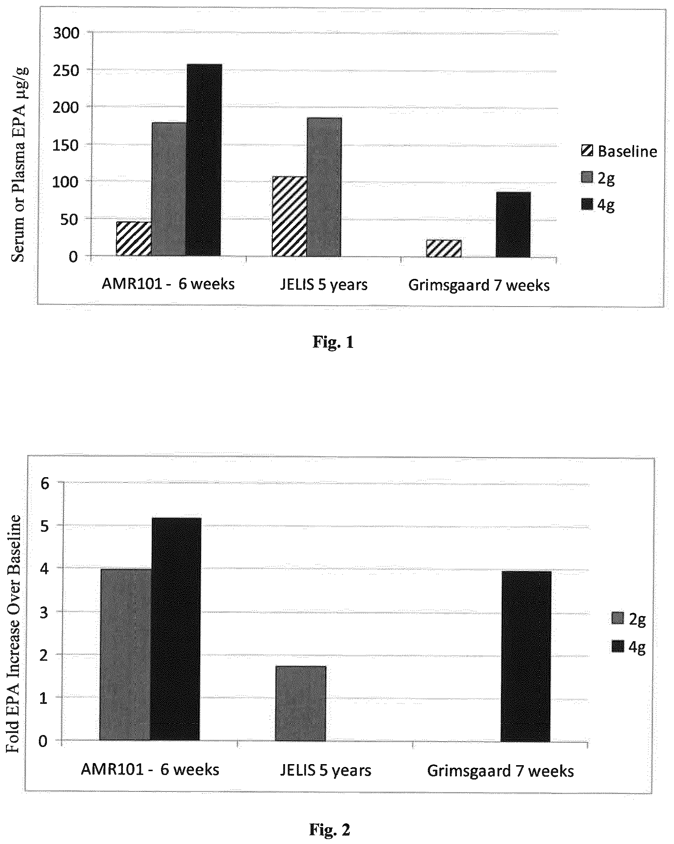

Figures 1 and 2 and show a comparison of the change in plasma/serum EPA levels observed with AMR101 treatment in the current study compared to those observed with different EPA compositions in the JELIS study and by Grimsgaard. As will be noted, at ~2 g per day, AMR101 achieved much greater plasma EPA increase compared to baseline (~4-fold) after just 6 weeks than the JELIS study observed (< 2-fold) after 5 years of treatment. Moreover, at the 4 g per day dose, AMR101 treatment for 6 weeks achieved much higher (>250 µg/g) plasma EPA levels than reported by Grimsgaard after 7 weeks of treatment (87.66 µg/g serum). Overall, the 4 g per day dose of AMR101 resulted in a greater than 5-fold increase in plasma EPA over baseline while the 4 g per day dose of Grimsgaard's composition resulted in less than a 3-fold increase in serum EPA. These results were unexpected.

### Example 2

A multi-center, randomized, double-blind, placebo-controlled trial was conducted in North America to determine whether 1 gram twice daily of EPA for 6 months improves motor performance in Huntington's patients. A post-hoc analysis was performed to evaluate the effect of EPA on non-fasting triacylglycerols.

Study of the effects of ethyl-EPA on the progression of Huntington Disease enrolled study participants at 41 sites in Canada and the United States. Based on the results of the earlier study, the study entry criteria were designed to enrich the participation of individuals with Huntington disease with a CAG repeat less than 45, without requiring genetic testing to reveal the length of expansions to research participants or investigators. To participate in the study, individuals had to have the clinical features of HD and either a confirmatory family history or a known CAG expansion. Eligibility criteria included a minimum age of 35, a total functional capacity of at least 7, minimal dystonia (not exceeding 2 on the UHDRS in either the trunk or extremities), minimal bradykinesia (not exceeding 2 on the UHDRS item for bradykinesia), the use of adequate birth control, the ability to take oral medications, and the willingness and ability to comply with study requirements. Individuals were not eligible to participate if, within 60 days of the baseline visit, they had used omega-3 fatty acid supplements, tetrabenazine or reserpine, high or variable doses of oral antipsychotic medications (e.g., haloperidol), steroids other than topical preparations, high dose selenium supplements, lithium, high doses of benzodiazepines, anti-coagulation medication (e.g., coumadin), high doses (greater than 325 mg per day) of aspirin, unstable does of NMDA receptor antagonists (e.g., memantine), unstable doses of anti-epileptic medications, or if they had participated in other investigational drug studies. Additional exclusion criteria were the use of depot neuroleptics within 6 months of the baseline visit, a history of tardive dyskinesia, unstable medical or psychiatric illness, major depression (defined as a score greater than 20 on the Beck Depression Inventory II), suicidal ideation, clinically significant substance abuse within 12 months of the baseline visit, women who were pregnant or lactating, known allergy to ethyl-EPA or placebo, or previous participation in an investigational study of EPA.

This was a randomized, double-blind, placebo-controlled, parallel group study of EPA (1 gram twice/day). The institutional review board at each participating site approved the research plan and consent documents. Eligible study participants provided written consent. At the baseline visit, participants were randomized according to a block-balanced computer-generated randomization plan that was stratified by site and generated by the Biostatistics Center at the University of Rochester. Individuals were randomized in a 1:1 ratio to receive either active drug (n=158) in the form of two 500 mg capsules of AMR101 orally or placebo (n=154) in the form of two 500 mg capsules containing light paraffin oil and 0.2% dl-alpha-tocopherol twice daily orally for 6 months. After 6 months, all TREND-HD participants were treated with AMR101 for 6 months in an open-label fashion. Only data from the first 6 months were used to evaluate the effects of AMR101 on lipids.

The outcome measure of this study was the change in non-fasting triacylglycerol (TG) levels in those on AMR101 compared to those on placebo.

Safety was assessed at all study visits, including evaluation and assessment of adverse events and serious adverse events and review of clinical laboratory tests (complete blood count, serum chemistry, and urine pregnancy tests). The safety of research participants was monitored in a blinded manner by a medical monitor from both the sponsor and from the Huntington Study Group. In addition, an independent Safety Monitoring Committee that had access to treatment assignments reviewed safety data throughout the study to determine if any modifications were needed to the trial's conduct.

Changes in lipid levels were compared using an analysis of covariance (ANCOVA) with treatment group as the factor of interest, site as a stratification factor, and baseline value as a covariate. All individuals who received study medication were included in the safety analysis. For each type of adverse event, the treatment groups were compared regarding the occurrence of at least one event using Fisher's exact test. Continuous measures of safety such as laboratory test results and vital signs were analyzed using methods similar to those described above for the primary outcome variable (ANCOVA). No corrections were made for multiple comparisons in evaluating safety data.

One hundred forty-five subjects on AMR101 (92% of those assigned) and 141 of those on placebo (92% of those assigned) had red blood cell content of EPA determined at baseline and 6 months. Baseline red blood cell content of 20:5n3 (EPA) increased significantly after 6 months in those on AMR101 (from a mean of 0.52% to 3.07%) but decreased in those on placebo (from a mean of 0.61% to 0.55%); p<0.0001). After 6 months, individuals taking AMR101 had a 26 mg/dL decrease in TGs from a baseline of 171 compared to a decrease of 11 mg/dL from a baseline of 187 mg/dL in those on placebo; p=0.007. Total cholesterol was reduced significantly more in those taking AMR101 (9.5 mg/dL) from a baseline of 204 mg/dL than in those taking placebo (2.5 mg/dL) from a baseline of 208 mg/dL; p=0.009. Lipid and Motor Scoer data are shown in Tables 5 and 6, respectively.

**Table 5. Motor Score Results.**

| | **All Study Participants n = 316** | | | **Study Participants with CAG < 45 n = 221** | | |
|---|---|---|---|---|---|---|
| **Total motor score 4 of the Unified Huntington's Disease Rating Scale** | **Ethyl-EPA** | **Placebo** | ***p-*value** | **Ethyl-EPA** | **Placebo** | ***p-*value** |
| At baseline [mean (SD)] | 25.2 (8.3) | 23.9 (8.1) | 0.16 | 24.9 (8.3) | 23.4 (7.7) | 0.18 |
| Change in total motor score 4 at 6 months (mean) | 0.2 | 1.0 | 0.20 | 0.0 | 0.3 | 0.70 |
| Change in total motor score 4 at 12 months (mean) | 0.0 | 2.0 | 0.02 | -1.2 | 1.6 | 0.004 |

**Table 6. Lipid Parameter Results.**

| **Lipoprotein Variable** | **Ethyl-EPA** | **Placebo** | ***p*-value** |
|---|---|---|---|
| Baseline triglycerides (mean mg/dL ± SD) | 171 ± 108 | 187 ± 139 | 0.27 |
| Baseline total cholesterol (mean mg/dL ± SD) | 204 ± 41.4 | 208 ± 40.6 | 0.42 |
| Change in triglycerides after 6 months (mean mg/dL ± SD) | -25.8 ± 89.1 | -11.1 ± 105.2 | .007 |
| Change in total cholesterol after 6 months (mean mg/dL ± SD) | -9.5 ± 28.6 | -2.5 ± 24.7 | .009 |
| Change in triglycerides after 12 months (mean mg/dL ± SD) | -17.7 ± 86.7 | -40.0 ± 126.0 | 0.66 |
| Change in total cholesterol after 12 months (mean mg/dL ± SD) | -5.6 ± 25.5 | -6.9 ± 34.5 | 0.95 |

By comparison with these data for AMR101, Grimsgaard reported a decrease (from baseline) of only 12% in serum triglycerides in the EPA group after 7 weeks of treatment. Furthermore, addition of the Epadel EPA composition to existing statin therapy in the JELIS study resulted in only a 9% reduction in triglycerides after 5 years of treatment.

### Example 3

A study was performed to evaluate and compare the content of Epadel capsules with AMR101 capsules. Six capsules of each composition were selected for analysis by gas chromatography. Averages of the six capsules for each of the two compositions are shown in Table 7.

**Table 7. Measured and Identified Components of AMR101 and Epadel.**

| **Component** | **AMR101** | **Epadel** |
|---|---|---|
| | **Amount (%w/w)** | |
| Ethyl-EPA | 96.3 | 94.5 |
| ODTA-E | 0.25 | 0.09 |
| Impurity 3 | ND | 0.06 |
| NDPA-E | 0.11 | 0.11 |
| Impurity 4 | 0.08 | 0.07 |
| AA-E | 0.30 | 0.06 |
| ETA-E | 0.38 | 0.11 |
| Isomer A | 0.08 | 0.23 |
| Isomer D,E | 0.11 | 0.62 |
| HPA-E | 0.11 | 0.06 |

| | | |
|---|---|---|
| ND = w/w% less than 0.05% | | |

### Example 4

A phase I, multiple dose pharmacokinetic study in healthy male volunteers was carried out at a single center. Twenty four subjects were divided into two treatment groups of 12 subjects each (groups A and B). Both groups received the same total daily dose of AMR101 but the dosing regiments were different. All subjects received a single oral dose of 2 g AMR101 on Day 1. Treatment Group A received 28 continuous once daily doses of 2 g AMR101. Treatment Group B received 27 continuous twice daily doses of 1 g AMR101 and a single does of 2 g of AMR101 on day 30.

Levels of EPA and other essential fatty acids were determined in plasma and red blood cells. Blood samples for pharmacokinetic analysis were taken at the following time points for Treatment groups A and B:
Days 1 and 30: Pre-dose, 1, 2, 3, 4, 5, 6, 8, 20, 12, 24, 36 and 48 h. post-dose;
Days 9, 16, 23: pre morning dose;
Days 37, 44, 58: post last dose.

A first Interim Report presents the following pharmacokinetic results for Treatment Group B:
Plasma - Day 1 (Pre-dose, 1, 2, 3, 4, 5, 6, 8, 20, 12, 24, 36 and 48 h post-dose);
Red cell-Day 1 (Pre-dose and 36 h), Day 30 (1 h post-dose), Day 37, Day 44, Day 58.

Using a corrected value obtained by subtracting the pre-administration concentration from the concentrations at each sampling, a single oral dose of 2 g of AMR101 resulted in a rapid rise in plasma lipid EPA. Maximum values were observed at 5 hours post-administration with EPA levels remaining above baseline at 48 hours post-administration. The half-life of removal of EPA from plasma lipids was 87 ± 65 h (non-baseline subtracted) and 42 ± 31 h (baseline subtracted). Summary pharmacokinetic data are shown in Table 8.

**Table 8. Non-Compartmental Analysis -Arithmetic Mean and SD.**

| | **Terminal Half-Life** | **Mean Residence Time (h)** | **Oral Clearance** | **VoD at Terminal Phase** | **VoD at Steady State** | **Max Drug Conc. (mg/ml)** | **Tmax (h)** |
|---|---|---|---|---|---|---|---|
| Unadjusted | 86.6 | 126.6 | 0.381 | 37.0 | 37.8 | 78.3 | 4.64 |
| SD | 65.4 | 93.3 | 0.202 | 13.2 | 13.5 | 33.7 | 0.92 |
| Baseline Subtracted | 42.2 | 63.6 | 1.27 | 58.8 | 62.8 | 55.5 | 4.64 |
| 0.021 | 30.9 | 43.1 | 0.83 | 23.9 | 25.7 | 28.2 | 0.92 |

In the Per Protocol population oral administration of AMR101 resulted in RBC EPA levels increasing from a mean value of 190.4 mg/g before dosing on Day 1 to 40.3 mg/g one hour following the final dose on Day 30.
Embodiments of the invention are described in the following numbered clauses.
1. A method of increasing plasma and/or serum EPA in a subject in need thereof, the method comprising administering, daily, to the subject a pharmaceutical composition comprising at least 96%, by weight, ethyl eicosapentaenoate, about 0.2% to about 0.5% by weight ethyl octadecatetraenoate, about 0.05% to about 0.25% by weight ethyl nonaecapentaenoate, about 0.2% to about 0.45% by weight ethyl arachidonate, about 0.3% to about 0.5% by weight ethyl eicosatetraenoate, about 0.05% to about 0.32% ethyl heneicosapentaenoate and not more than 0.05% ethyl-DHA, if any, in an amount sufficient to increase plasma and/or serum EPA levels in the subject by at least about 200% to compared to baseline.
2. The method of clause 1 wherein the composition is administered in an amount sufficient to increase plasma and/or serum EPA levels in the subject by at least 300% compared to baseline.
3. The method of clause 1 wherein the composition is administered in an amount sufficient to increase plasma and/or serum EPA levels in the subject by at least 400% compared to baseline.
4. The method of clause 3 wherein the composition is administered to the subject daily for a period of at least about 6 weeks.
5. The method of clause 4 wherein the subject has a baseline plasma and/or serum EPA level of not more than about 50 µg/ml.
6. The method of clause 1 wherein upon administration of 2 g of the composition to the subject daily for a period of at least about 6 weeks, the subject exhibits an at least 200% increase in plasma and/or serum EPA compared to baseline.
7. The method of clause 1 wherein upon administration of 4 g of the composition to the subject daily for a period of at least about 6 weeks, the subject exhibits an at least 300% increase in plasma and/or serum EPA compared to baseline.
8. The method of clause 1 wherein upon daily administration of 4 g of the composition to the subject for a period of at least about 6 weeks, the subject exhibits an at least 400% increase in plasma and/or serum EPA compared to baseline.
9. The method of clause 1 wherein upon daily administration of the composition to the subject, the subject exhibits an increase in serum and/or plasma DPA levels and a decrease in serum and/or plasma AA, DHA and/or DGLA levels.
10. The method of clause 1 wherein upon daily administration of the composition to the subject for a period of at least 6 weeks, the subject exhibits an increase in serum and/or plasma DPA levels and a decrease in serum and/or plasma AA, DHA and/or DGLA levels compared to baseline.
11. The method of clause 1 wherein upon daily administration of the composition to the subject for a period of at least 6 weeks, the subject exhibits a decrease in plasma and/or serum DHA levels by at least 16% compared to baseline, a decrease in DGLA plasma and/or serum levels by at least 31% compared to baseline, a decrease in AA plasma and/or serum levels by at least 20% compared to baseline, and/or an increase in plasma and/or serum DPA levels of at least 130% compared to baseline.
12. The method of clause 1 wherein the composition comprises at least 96% by weight ethyl eicosapentaenoate, about 0.22% to about 0.4% by weight ethyl octadecatetraenoate, about 0.075% to about 0.20% by weight ethyl nonaecapentaenoate, about 0.25% to about 0.40% by weight ethyl arachidonate, about 0.3% to about 0.4% by weight ethyl eicosatetraenoate and about 0.075% to about 0.25% ethyl heneicosapentaenoate.
13. The method of clause 1 wherein the composition comprises at least 98%, by weight, ethyl eicosapentaenoate, about 0.25% to about 0.38% by weight ethyl octadecatetraenoate, about 0.10% to about 0.15% by weight ethyl nonaecapentaenoate, about 0.25% to about 0.35% by weight ethyl arachidonate, about 0.31% to about 0.38% by weight ethyl eicosatetraenoate, and about 0.08% to about 0.20% ethyl heneicosapentaenoate.
14. The method of clause 1 wherein the composition further comprises tocopherol in an amount of about 0.1% to about 0.3%, by weight.
15. The method of clause 1 wherein the composition is present in a capsule shell.
16. A method of increasing plasma and/or serum EPA in a subject in need thereof, the method comprising administering to the subject a pharmaceutical composition comprising at least 96%, by weight, ethyl eicosapentaenoate, about 0.2% to about 0.5% by weight ethyl octadecatetraenoate, about 0.05% to about 0.25% by weight ethyl nonaecapentaenoate, about 0.2% to about 0.45% by weight ethyl arachidonate, about 0.3% to about 0.5% by weight ethyl eicosatetraenoate, about 0.05% to about 0.32% ethyl heneicosapentaenoate, and not more than 0.05% ethyl-DHA, wherein upon daily administration of 2 g of the composition to the subject for a period of at least about 6 weeks, the subject exhibits an at least about 200% increase in plasma and/or serum EPA compared to baseline.
17. The method of clause 15 wherein the subject has a baseline plasma or serum EPA level of not more than about 50 µg/ml.
18. A method of increasing plasma or serum EPA in a subject in need thereof, the method comprising administering to the subject a pharmaceutical composition comprising at least 96%, by weight, ethyl eicosapentaenoate, about 0.2% to about 0.5% by weight ethyl octadecatetraenoate, about 0.05% to about 0.25% by weight ethyl nonaecapentaenoate, about 0.2% to about 0.45% by weight ethyl arachidonate, about 0.3% to about 0.5% by weight ethyl eicosatetraenoate, about 0.05% to about 0.32% ethyl heneicosapentaenoate, and not more than 0.05% ethyl-DHA, wherein upon daily administration of 4 g of the composition to the subject for a period of at least about 6 weeks, the subject exhibits an at least about 300% increase in plasma and/or serum EPA compared to baseline.
19. The method of clause 18 wherein the subject has a baseline plasma or serum EPA level of not more than about 50 µg/ml.
20. A method of maintaining LDL control in a subject who is on stable statin therapy and requires triglyceride lowering therapy, the method comprising identifying a subject who is on stable statin therapy and requires triglyceride lowering therapy, administering to the subject a pharmaceutically acceptable composition comprising about 1 g to about 4 g of ultra-pure E-EPA per day, wherein upon administering the composition to the subject, the subject exhibits a clinically significant reduction in fasting triglycerides compared to control.
21. A pharmaceutical composition comprising about 1 g to about 4 g of ultra-pure E-EPA for use in maintaining LDL control in a subject who is on stable statin therapy and requires triglyceride lowering therapy, wherein upon administering the composition to the subject, the subject exhibits a clinically significant reduction in fasting triglycerides compared to control.

## Claims

1. A pharmaceutical composition comprising about 1 g to about 4 g of at least 96%, by weight of all fatty acids present in the composition, ethyl eicosapentaenoate, for use in maintaining LDL control in a subject who is on stable statin therapy and requires triglyceride lowering therapy, wherein upon administering the pharmaceutical composition to the subject, the subject exhibits a clinically significant reduction in fasting triglycerides compared to control.

2. The pharmaceutical composition for the use according to claim 1, wherein the clinically significant reduction in fasting triglycerides compared to control is a reduction of 10 mg/dl.

3. The pharmaceutical composition for the use according to any of claims 1 to 2, comprising at least 96% by weight ethyl eicosapentaenoate (EPA-E), about 0.2% to about 0.5% by weight ethyl octadecatetraenoate (ODTA-E), about 0.05% to about 0.25% by weight ethyl nonaecapentaenoate (NDPA-E), about 0.2% to about 0.45% by weight ethyl arachidonate (AA-E), about 0.3% to about 0.5% by weight ethyl eicosatetraenoate (ETA-E), and about 0.05% to about 0.32% ethyl heneicosapentaenoate (HPA-E).

4. The pharmaceutical composition for the use according to any of claims 1 to 3, comprising not more than about 0.06% by weight docosahexaenoic acid (DHA) or derivative thereof such as ethyl-DHA.

5. The pharmaceutical composition for the use according to any of claims 1 to 4, wherein the pharmaceutical composition is administered to provide to the subject about 1 g to about 4 g of at least 96%, by weight of all fatty acids present in the composition, ethyl eicosapentaenoate, per day..

6. The pharmaceutical composition for the use according to any of claims 1 to 5, wherein the pharmaceutical composition further comprises tocopherol in an amount of about 0.05% to about 0.4%, , by weight.

7. The pharmaceutical composition for the use according to any of claims 1 to 6, wherein the pharmaceutical composition is present in a capsule shell.
